# EUROPEAN PATENT APPLICATION

(11) **EP 4 742 257 A1**
(43) Date of publication of application: **13.05.2026**
(21) Application number: 25815235.4
(22) Date of filing: 27.05.2025
(51) Int. Cl.: G16H 10/60, G16H 50/20, A61B 5/00

(54) **LIFE INFORMATION PROCESSING SYSTEM AND LIFE INFORMATION PROCESSING METHOD**

(30) Priority: 27.05.2024 WO PCT/CN2024/095615
(71) Applicant: Shenzhen Mindray Bio-Medical Electronics Co., Ltd., Shenzhen, Guangdong 518057 (CN)
(72) Inventor: TAN, Lin, Shenzhen, Guangdong 518057 (CN); ZOU, Xiaoling, Shenzhen, Guangdong 518057 (CN); XIAO, Ke, Shenzhen, Guangdong 518057 (CN); GUAN, Zehong, Shenzhen, Guangdong 518057 (CN); YU, Zedan, Shenzhen, Guangdong 518057 (CN); JIN, Xingliang, Shenzhen, Guangdong 518057 (CN)
(74) Representative: KIPA AB
(86) International application number: PCT/CN2025/097598
(87) International publication number: WO 2025/247244

(57) **Abstract**

A system and method for processing vital information are disclosed, including: obtaining patient data of a patient; analyzing patient data to obtain an abnormal state of the patient and a waveform of the patient data associated with the abnormal state of the patient, wherein the waveform of the patient data has an abnormal waveform segment associated with the abnormal state of the patient, which includes at least one abnormal state of a physiological structure of the patient; the physiological structure includes at least one of a nervous system, a circulatory system, and a respiratory system; displaying state information and the waveform of the patient data representing the abnormal state of the patient on a display interface, and providing graphic and text for explaining the abnormal waveform segment. Through the system and method, user can have a more comprehensive and rapid understanding of the abnormal state of the patient.

## Description

### TECHNICAL FIELD

The disclosure relates to a technical field of medical technology, and more particularly to a system for processing vital information and a method for processing vital information.

### BACKGROUND

At present, when medical staff understand and consider a condition of a patient, they need to select various types of data from various monitoring parameters to synthetically consider the condition of the patient. In order to gather these data, medical staff need to search for these data on different devices or information systems, which is very time-consuming. Especially when a condition of a patient is abnormal, medical staff often cannot intuitively view data associated with an abnormality, and even do not know what rules to follow in order to better understand an abnormal condition of the patient.

### SUMMARY

This disclosure mainly provides a system for processing vital information and a method for processing vital information that can help a user to better understand an abnormal state of a patient.

According to a first aspect, an embodiment provides a method for processing vital information, including:
obtaining patient data from a patient, wherein the patient data includes data corresponding to multiple clinical parameters, which data are associated with a physiological structure and obtained within a preset time range; wherein the physiological structure is pre-associated with one or more rules, each rule is pre-associated with one abnormal state of the physiological structure, wherein each rule includes: multiple abnormal indicators that correspond to the multiple clinical parameters; the physiological structure at least includes a physiological system, wherein the physiological system includes at least one of: a nervous system, a circulatory system or a respiratory system;
analyzing data that correspond to several of the multiple clinical parameters and determining a target rule; wherein the target rule includes the several clinical parameters analyzed; wherein data, which correspond to each of the several clinical parameters that is included in the target rule, have reached an abnormal indicator that corresponds to said each clinical parameter; wherein an abnormal indicator, which corresponds to at least one of the several clinical parameters analyzed that is included in the target rule, is at least associated with a trend of change for said at least one clinical parameter;
based on the data that correspond to each of the several clinical parameters that is included in the target rule, obtaining a waveform of the data corresponding to said each clinical parameter; and determining a portion of the waveform of the data corresponding said each clinical parameter as an abnormal waveform segment, wherein the portion of the waveform of the data corresponding to said each clinical parameter reaches the abnormal indicator corresponding to said each clinical parameter; and
controlling a display interface of a display to display the abnormal state of the physiological structure that corresponds to the target rule, and to display the waveform of the data corresponding to said each clinical parameter that is included in the target rule; wherein for each waveform, further displaying the corresponding abnormal indicator in a form of graphic and/or text on the abnormal waveform segment of said waveform or at an adjacent position of the abnormal waveform segment of said waveform.

According to a second aspect, an embodiment provides a method for processing vital information, including:
obtaining patient data from a patient;
analyzing the patient data to obtain an abnormal state of the patient and a waveform of the patient data that are associated with the abnormal state of the patient; wherein the waveform of the patient data has an abnormal waveform segment that is associated with the abnormal state of the patient; the abnormal state of the patient includes an abnormal state of at least one physiological structure of the patient, wherein the physiological structure at least includes a physiological system, wherein the physiological system includes at least one of a nervous system, a circulatory system or a respiratory system, of the patient; and
controlling a display interface to display state information that represents the abnormal state of the patient, and the waveform of the patient data, wherein the state information includes graphic and text that explain the abnormal waveform segment; wherein displaying specifically includes associatively displaying the graphic and the text with the abnormal waveform segment, wherein the graphic at least includes an arrow that represents a trend of change for the patient data; wherein associatively displaying the graphic and the text with the abnormal waveform segment, includes: displaying the arrow on or adjacent to the abnormal waveform segment; wherein:
when the physiological system includes the nervous system of the patient, the text is configured to describe an abnormal state of an indicator that is associated with a brain nerve;
when the physiological system includes the circulatory system of the patient, the text is configured to describe an abnormal state of an indicator that is associated with hemodynamics or perfusion;
when the physiological system includes the respiratory system of the patient, the text is configured to describe an abnormal state of an indicator that is associated with oxygenation.

According to a third aspect, an embodiment provides a method for processing vital information, including:
obtaining patient data of a patient;
analyzing the patient data to obtain an abnormal state of the patient and a waveform of the patient data that are associated with the abnormal state of the patient; wherein the waveform of the patient data has an abnormal waveform segment that is associated with the abnormal state of the patient; and
controlling a display interface to display state information that represents the abnormal state of the patient, and the waveform of the patient data, and providing graphic and text that explain the abnormal waveform segment; wherein the graphic and the text are associatively displayed with the abnormal waveform segment.

According to a fourth aspect, an embodiment provides a system for processing vital information, including: a memory, a processor and a display, wherein the memory is configured to store an executable program, the processor is configured to execute the executable program, so as to enable the processor to implement the methods described above.

According to a fifth aspect, an embodiment provides a computer-readable storage medium, including a program that is executable by a processor to implement the method described above.

According to above embodiments of a system for processing vital information and a method for processing vital information, after obtaining patient data, the patient data is analyzed to obtain an abnormal state of a patient and a waveform of the patient data that are associated with the abnormal state, and graphic and text are provided on a display interface to explain an abnormal waveform segment in the waveform of the patient data, so as to enable a user to intuitively and centrally understand the patient data associated with the abnormal state, why the abnormal state occurs, and other information, thereby more comprehensively and accurately understanding the abnormal state of the patient.

### BRIEF DESCRIPTION OF THE DRAWINGS

FIG. 1 is a block diagram of a system for processing vital information according to an embodiment.
FIG. 2 is a flowchart of a method for processing vital information according to an embodiment.
FIG. 3 is a diagram of a display interface of a system for processing vital information according to an embodiment.
FIG. 4 is a diagram of a display interface of a system for processing vital information according to another embodiment.
FIG. 5 is a diagram of a display interface of a system for processing vital information according to another further embodiment.
FIG. 6 is a diagram of a display interface of a system for processing vital information according to another further embodiment.
FIG. 7 is a flowchart of a method for processing vital information according to another embodiment.

### DESCRIPTION OF THE PREFERRED EMBODIMENTS

This disclosure will be further described in detail through specific implementation methods combined with the accompanying drawings as follows. Similar elements in different implementation methods are labeled with corresponding element numbers. In the following embodiments, many details are described to facilitate a better understanding of this disclosure. However, technicians in this field can easily recognize that some features can be omitted or replaced by other components, materials, or methods in different situations. In some cases, some operations related to this disclosure are not displayed or described in the description, in order to avoid the core part of this disclosure being overwhelmed by excessive description. For those skilled in the art, a detailed description of these related operations is not necessary, and they can fully understand the relevant operations based on the description in this disclosure and general technical knowledge in the field.

In addition, the features, operations, or characteristics described in this disclosure can be combined in any appropriate way to form various implementation methods. Meanwhile, orders of steps or actions described in the method description can also be changed or adjusted in a way that is obvious to those skilled in the art. Therefore, the various orders in the description and drawings are only for a purpose of clearly describing a certain embodiment and do not necessarily mean that they are necessary orders, unless otherwise specified that one of the orders must be followed.

A numbering for components in this disclosure, such as "first", "second", etc., is only used to distinguish the described objects and does not have any order or technical meaning. The terms "connection" and "linkage" referred to in this disclosure, unless otherwise specified, include both direct and indirect connections (linkages).

A waveform referred to in this disclosure includes a real-time waveform, a trend waveform (trend chart), a portion of a real-time waveform, a portion of a trend waveform, etc.

At present, when a patient experiences an abnormal state, medical staff often view and analyze relevant patient data on different apparatuses or devices based on their experience, in order to analyze reason(s) for the abnormal state of the patient. This can be time-consuming and laborious for medical staff, who may also miss some important patient data. The most important idea of this disclosure is to display the waveform of the patient data associated with the abnormal state of the patient, as well as graphic and text used to explain an abnormal waveform segment in the waveform of the patient data, when obtaining the abnormal state of the patient, so as to enable the user to comprehensively and quickly grasp a specific situation of the abnormal state.

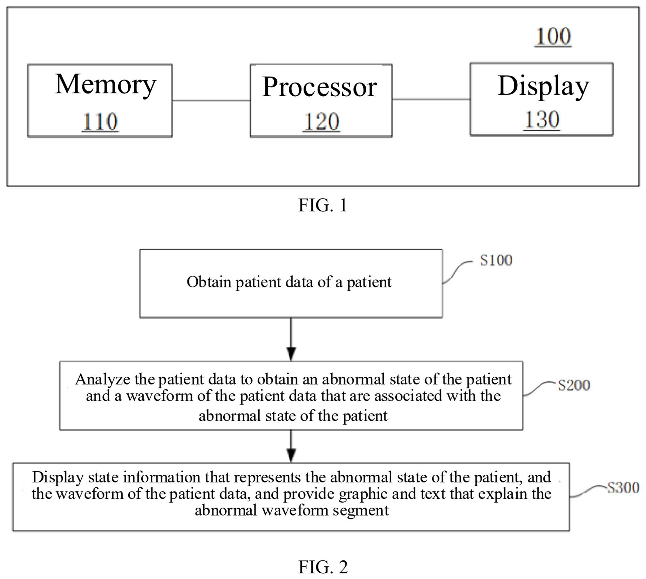

Referring to FIG. 1, the system for processing vital information 100 of an embodiment includes a memory 110, a processor 120, and a display 130, wherein the memory 110 is configured to store an executable program, and the processor 120 is configured to execute the executable program stored in the memory 110.

The system for processing vital information 100 of this embodiment includes but is not limited to any one or a combination of: a monitor, a local central station, a remote central station, a cloud service system, and a mobile terminal. The system for processing vital information 100 can be a portable system for processing vital information, a transportable system for processing vital information, or a mobile system for processing vital information.

In an embodiment, the system for processing vital information 100 can be a monitor used for real-time monitoring of monitoring parameters of a patient, and can include a bedside monitor, a wearable monitor, etc. The monitor can include a ventilator monitor, an anesthesia monitor, a defibrillator monitor, an intracranial pressure monitor, an electrocardiogram monitor, etc.

The system for processing vital information 100 may also include a central station for receiving monitoring data transmitted by the monitor and centrally monitoring the monitoring data. Wherein, the central station can include a local central station or a remote central station. The central station is connected with monitors within one or multiple departments through a network to achieve real-time centralized monitoring and massive data storage. For example, the central station stores monitoring data, basic patient information, medical history information, and diagnostic information, but is not limited to these.

In some embodiments, the monitor and the central station can form an interconnected platform through BeneLink to achieve data communication between the monitor and the central station. For example, the central station can access the monitoring data monitored by the monitor. In other embodiments, the monitor and the central station can also establish a data connection through a communication unit, which includes but is not limited to a 2G, 3G, 4G, 5G communication unit, such as Wi-Fi, Bluetooth, or mobile communication.

The system for processing vital information 100 of this embodiment can also include another device besides a monitoring device, such as an image acquisition device, a treatment and support device, an information system (such as CIS, HIS, shift handover software, decision support system, etc.), a mobile terminal (such as RV inspection), etc.

The processor 120 of the system for processing vital information 100 can be a central processing unit (CPU), as well as another general-purpose processor, a digital signal processor (DSP), an application specific integrated circuit (ASIC), a field programmable gate array (FPGA), or another programmable logic device, a discrete gate or transistor logic device, a discrete hardware component, etc. A general-purpose processor can be a microprocessor or any conventional processor. The processor 120 is a control center of the system for processing vital information 100, and is connected with various parts of the entire system for processing vital information 100 through various interfaces and circuits.

The memory 110 of the system for processing vital information 100 is configured to store an executable program. For example, the memory 110 may mainly include a program storage region and a data storage region, wherein the program storage region may store an operating system, application programs required for multiple functions, etc. In addition, the memory 110 may include a highspeed random access memory, as well as a non-volatile memory, such as a hard drive, a memory, a plug-in hard drive, a smart storage card, a secure digital card, a flash memory card, multiple disk storage devices, multiple flash memory devices, or other volatile solid-state storage devices.

The display 130 is configured to provide a visual display output to a user. Specifically, the display 130 can be used to provide a visual display interface for a user, including but not limited to a monitoring interface, an interface for setting a monitoring parameter, etc. For example, the display 130 can be implemented as a touch display, or a display 130 with an input panel, i.e., the display 130 can serve as an input/output device.

In some embodiments, the system for processing vital information 100 further includes a data acquisition unit, such as a sensor. The sensor can be used to continuously acquire monitoring data from a patient. Wherein, continuously acquiring refers to that the sensor continuously measures monitoring data for multiple times with a preset time interval, wherein the preset time interval refers to a shortest time for the sensor to return monitoring data once. The data acquisition unit and the processor 120 can be connected through wired or wireless communication protocols, so as to realize data exchange between the data acquisition unit and the processor 120. Wireless communication technologies include but are not limited to: various generations of mobile communication technologies (2G, 3G, 4G, and 5G), various wireless networks, Bluetooth, ZigBee, ultra-wideband UWB, NFC, etc.

The system for processing vital information 100 may also include a communication unit that is connected with the processor 120. In some embodiments, the system for processing vital information 100 may establish data communication with a third-party device through a communication unit. The processor 120 also controls the communication unit to obtain data from the third-party device, or transmit the patient data collected by the data acquisition unit to the third-party device. The communication unit includes but is not limited to a Wi-Fi communication unit, a Bluetooth communication unit, an NFC communication unit, a ZigBee communication unit, a Ultra-wideband (UWB) communication unit, or a mobile communication unit, such as 2G, 3G, 4G, 5G, etc. In other embodiments, the system for processing vital information 100 can also establish a connection with a third-party device through a cable. The third-party device includes but is not limited to a ventilator, an anesthesia machine, an infusion pump, an image acquisition device, and another medical device. The third-party device can also be a cloud service system or a non-medical device, such as a mobile phone, a tablet, and a personal computer.

In some embodiments, the system for processing vital information 100 further includes an alarm unit that is connected with the processor 120 for outputting an alarm prompt for medical staff to perform a corresponding rescue measure. The alarm unit includes but is not limited to an alarm light, an alarm speaker, etc. The alarm information can also be displayed on the monitor 130, so as to alarm the medical staff by flashing the alarm light, or being played through the alarm speaker.

In order to achieve user interface and data exchange, in addition to the display 130, the system for processing vital information 100 may also include another input/output device that is connected with the processor 120, including but not limited to an input device, such as a keyboard, a mice, a touch screen, a remote control, as well as an output device, such as a printer and a speaker.

It should be understood that, FIG. 1 is only an example of components included in a system for processing vital information 100 and does not constitute a limitation of the system for processing vital information 100. The monitoring device 100 may include more or fewer components than shown in FIG. 1, or combine certain components or different components.

Based on the above system for processing vital information 100, as shown in FIG. 2, some embodiments provide an information processing method, including:
Step S100, obtain patient data from a patient.

There are multiple ways to obtain patient data, for example, one way is to obtain patient data of real-time monitoring, and the other way is to obtain patient data during a monitoring period for a patient state for one time. In addition to the patient data obtained by the system for processing vital information 100 itself, the system for processing vital information 100 can also obtain other patient data from an external medical device or a non-medical device. For example, the external device includes a treatment device, an examination device, and a third-party system, wherein the treatment device includes a ventilator, an anesthesia machine, an infusion pump, an extracorporeal circulation device, etc.; the examination device includes an ultrasound imaging device, an endoscopic device, etc.; and the third-party system includes a PACS system Picture Archiving and Communication System), an LIS system (Laboratory Information System), a CIS system (Clinical Information System), etc. For example, the patient data obtained from the external device include at least one of: monitoring data and/or device data collected by a ventilator device, monitoring data and/or device data collected by an anesthesia machine, monitoring data and/or device data collected by an infusion pump device, medical condition data, test data, and examination data. The system for processing vital information 100 can obtain the above patient data through communication connection with the external device.

For example, the medical condition data includes at least one of: patient basic information, disease diagnosis data, treatment data, nursing data, or electronic medical record data. Wherein, the patient basic information includes age, weight, gender, etc., and the disease diagnosis data includes a medical history, a diagnosis report, a medical advice, a consultation dialogue, etc. The system for processing vital information 100 can obtain the medical condition data through an electronic medical record or receive the medical condition data inputted by medical staff. The test data includes at least one of: blood routine test data, liver function test data, kidney function test data, thyroid test data, urine test data, immune test data, coagulation test data, blood gas test data, stool routine test data, and tumor marker test data, which test data are collected by in-vitro diagnostic device. The system for processing vital information 100 can obtain the test data of the patient through a hospital laboratory information management system. The examination data includes data collected by a medical imaging device, specifically including at least one of: DR imaging data, CT imaging data, MRI imaging data, PET imaging data, ultrasound imaging data, scale data, and physical examination data. The system for processing vital information 100 can obtain the examination data from a medical imaging device or an imaging induction and communication system.

The patient data obtained can be medical data within a certain time range, which can be a preset time range, such as 24 hours, or can be a suitable time range preset according to a user instruction. Specifically, the patient data within a certain time range can be selected based on a timestamp of the medical data. If there are no certain types of patient data within this time range, for example, some laboratory test data may not have test results within 24 hours, the most recently obtained patient data of that type can be selected for subsequent data processing.

For some patient data, especially physiological data obtained by a data acquisition device of the system for processing vital information 100, preprocessing can also be performed, including filtering, abnormal/null value processing, data sampling alignment, and other operations. Data sampling alignment refers to an interpolation operation of intermittent measurement data, such as blood pressure, so as to ensure synchronous analysis of intermittent measurement data with other data.

Preprocessing patient data can extract patient data that satisfy a quality requirement for subsequent analysis, especially for continuous measurement data in patient data. Quality analysis can help filter out data that are generated by interference and has no actual clinical value. The patient data obtained by the system for processing vital information 100 can present various aspects of patient information, but it can also be seen from relevant descriptions that there are various types of patient data associated with the patient, and a large part of the data constantly changes over time. It is difficult for clinical medical staff to comprehensively and efficiently assess the above patient data and make decisions based on the above patient data. Therefore, the system for processing vital information 100 of this embodiment automatically determines the patient state based on the patient data, in order to comprehensively utilize the patient data and quickly extract effective information from the patient data.

Step S200, analyze the patient data to obtain an abnormal state of the patient and a waveform of the patient data that are associated with the abnormal state of the patient.

A patient state for a patient is an assessment of an overall physiological function or a partial physiological function of the patient, reflecting an overall health start or a partial health state of the patient. The patient state can be at least one of: an overall state, a physiological system state, an organ state, a physiological part state, or a tissue state, of the patient. The physiological system state, the organ state or the tissue state reflects a macroscopic state of a corresponding system, organ, or tissue. Wherein the physiological system includes at least one of: a motor system, a nervous system, an endocrine system, a circulatory system, a respiratory system, a digestive system, a urinary system, or a reproductive system. The physiological organ includes at least one of: brain, heart, lungs, liver, stomach, or kidneys. The physiological part includes at least one of: head, chest, or abdomen. The tissue includes at least one of: a muscle tissue, a nerve tissue, or an epithelial tissue. A feature of the physiological system or a feature of the organ includes coagulation, nutrition, infection, or blood sugar.

In some embodiments, by integrating and extracting information contained in multiple patient data, the patient data can be matched and determined based on a rule library, so as to determine a patient state. The rule library contains a large number of preset rules, and a pre-established correspondence exist between preset rules and patient states. The correspondence includes: each preset rule corresponds to one patient state, that is, if one preset rule is satisfied, the patient is determined to have one patient state corresponding to said one preset rule. Alternatively, each preset rule corresponds to multiple patient states, meaning that as long as one preset rule is satisfied, the patient has been determined to have multiple patient states simultaneously. Alternatively, multiple preset rules correspond to one patient state, meaning that only when multiple preset rules are satisfied simultaneously, the patient can be determined to have said one patient state corresponding to those preset rules. The preset rule can be formulated based on various methods, such as a guideline rule, a clinical consensus, and a clinical research. Compared with determining the patient state based on a machine learning model, determining a patient state based on a preset rule has a higher accuracy, a more controllable result, and is more in line with a clinical cognition of medical staff. One or more preset rules can be stored locally or on a server. These preset rules include target rule(s) corresponding to abnormal state(s). In this step, the patient data associated with the patient state can be analyzed to determine one or more target rules that are satisfied by the patient data, and obtain the abnormal state of the patient corresponding to the one or more target rules that are satisfied by the patient data.

For example, a single type of patient data can be analyzed, or at least two types of patient data can be analyzed. When analyzing at least two types of patient data, same, similar, or adjacent patient data can be selected for analysis based on time information carried in the patient data. Analyzing at least two types of patient data can include calculating respective maximums, respective minimums, respective averages, or a correlation between the at least two types of patient data. For example, when calculating a correlation between a heart rate and a blood pressure, a suitable correlation calculation method, such as Pearson and Kendall can be selected, and respective start time and respective end time of heart rate data and blood pressure data can be preset. The respective start time and respective end time of the heart rate data and the blood pressure data can be exactly the same or have some differences.

In some embodiments, one target rule includes one or more indicators, determining whether the patient data satisfy one target rule is to determine whether the patient data satisfy one or more indicators in the target rule. If the patient data satisfy one or more indicators in the target rule, then the patient data satisfy the target rule. Conversely, if the patient data do not satisfy one or more indicators in the target rule, then the patient data do not satisfy the target rule. For example, if three indicators are included in the target rule, and the patient data satisfy these three indicators, then the patient data satisfy the target rule. For example, if the target rule has three indicators, and the patient data satisfy two or more of these indicators, then the patient data satisfy the target rule.

For example, a target rule that is associated with a nervous system of the patient include an indicator that is associated with a brain nerve, a target rule that is associated with a circulatory system of the patient includes an indicator that is associated with hemodynamics or perfusion, and a target rule that is associated with a respiratory system of the patient include an indicator that is associated with oxygenation. When determining the abnormal state of the nervous system of the patient, the patient data that are associated with the brain nerve can be selected, and an indicator, which is associated with the brain nerve and satisfied by the patient data, can be determined. When determining the abnormal state of the circulatory system of the patient, the patient data that are associated with hemodynamics or perfusion can be selected, and an indicator which is associated with hemodynamics or perfusion and satisfied by the patient data, can be determined. For example, by simultaneously detecting abnormalities, such as a trend of decrease in perfusion and a trend of decrease in blood pressure in a patient, the patient can be determined to be at risk of shock. The perfusion can be determined by a PI parameter (perfusion index) or a CQI parameter (cardiopulmonary resuscitation quality index). When determining the abnormal state of the respiratory system of the patient, patient data that are associated with oxygenation can be selected, and an indicator, which is associated with oxygenation and satisfied by the patient data, can be determined.

An indicator in the above target rule includes but is not limited to an indicator that is associated with a threshold, an indicator that is associated with a trend of change, an indicator that is associated with a waveform shape, etc. Below are examples to illustrate.

For the indicator that is associated with a threshold, a first comparison result between the patient data and a preset threshold can be obtained, and whether the patient data satisfy one or more indicators in the target rule can be determined based on the first comparison result. For example, if an indicator is "average heart rate greater than 90", an average heart rate of a patient is obtained and compared to 90. If the average heart rate is greater than 90, the indicator is satisfied, otherwise the indicator is not satisfied. In some cases, the indicator that is associated with a threshold is also accompanied by a time condition. For example, one indicator in the target rule is "a number of times for an average heart rate being greater than 90 is greater than 8 times". For this indicator, the average heart rate of the patient in the past 4 hours is obtained to determine whether the number of times for the average heart rate of the patient being greater than 90 is greater than 8 times in the past 4 hours. If it is, then this indicator is satisfied; otherwise, the indicator is not satisfied.

For the indicator that is associated with a trend of change, the trend of change for the patient data can be obtained to determine whether the patient data satisfy one or more indicators in the target rule based on the trend of change. The trend of change includes but is not limited to at least one or a combination of: an upward trend of change, a downward trend of change, a fluctuated trend of change, and an abrupt trend of change, such as a trend of change which is first upward and then downward, or the trend of change which is first downward and then downward.

For example, one indicator is "heart rate continuously increases for more than 4 hours". For this indicator, the change in the heart rate of the patient is obtained. If the heart rate of the patient continuously increases for more than 4 hours over a time period, this indicator is satisfied; otherwise, the indicator is not satisfied. In some cases, a single indicator may also include respective trends of change for more than one types of patient data. Analyzing and determining multiple trends of change are more in line with physiological laws and help improving an accuracy of identifying the abnormal state in the patient. In addition, for different types of patient data, durations corresponding to indicators can also be different, or for a same type of patient data, durations corresponding to indicators may also be different under a same trend of change but different rates of change.

For example, a first clinical parameter is ECG, and its corresponding first indicator is heart rate value, a first trend of change in the target rule is a trend of change for the heart rate value (hereinafter referred to as trend of change for heart rate); a second clinical parameter is Spo2, and its corresponding second indicator is blood oxygen value, a second trend of change in the target rule is a trend of change for the blood oxygen value (hereinafter referred to as trend of change for blood oxygen). After extracting the heart rate value from the heart rate data, the trend of change for the heart rate value in a first time range is determined, such as determining that the heart rate value shows an upward trend in the first time range. And, after extracting the blood oxygen value from the blood oxygen data, determine the trend of change for the blood oxygen value in the second time range, such as determining that the blood oxygen value shows a downward trend in the second time range. Wherein, the first time range and the second time range can be completely identical, at least partially identical, or adjacent to each other. There are various ways to determine the trend of change. Taking the trend of change for heart rate as an example, a linear fitting can be performed on heart rate values within the first time range, and the trend of change for heart rate can be determined based on a slope of the fitted line. Alternatively, the heart rate values within different time windows can be averaged, and the trend of change for heart rate can be determined by comparing respective magnitudes of the average values. This embodiment does not limit a calculation method for the trend of change.

After obtaining the trend of change for heart rate and the trend of change for blood oxygen, the trend of change for heart rate and the trend of change for blood oxygen can be compared with abnormal indicators in the target rule to determine whether a combined change of heart rate and blood oxygen can reflect a certain patient state. Wherein, the abnormal indicators used for comparing with the trend of change for heart rate and the trend of change for blood oxygen at least define a preset trend of change for heart rate and a preset trend of change for blood oxygen. Comparing the trend of change for heart rate and the trend of change for blood oxygen at least includes determining whether an actual trend of change for heart rate is consistent with the preset trend of change for heart rate, and whether an actual trend of change for blood oxygen is consistent with the preset trend of change for blood oxygen. Data of the patient (such as physiological parameters and other data) rarely change individually, but mostly undergo collaborative changes. Analyzing and determining multiple trends of changes are more in line with physiological laws and help improving an accuracy for identifying a patient state.

For example, in a preset rule that is associated with a circulatory deterioration, when the preset trend of change for heart rate is an upward trend of change and the preset trend of change for blood oxygen is a downward trend of change, then if an actual trend of change for heart rate is an upward trend and an actual trend of change for blood oxygen is a downward trend, then the preset rule is satisfied. If the actual trend of change for heart rate and the actual trend of change for blood oxygen are both an upward trend of change or a downward trend of change, or if the trend of change for heart rate is a downward trend of change and the trend of change for blood oxygen is an upward trend of change, then the preset rule is not satisfied. Other combinations of the trend of change for heart rate and the trend of change for blood oxygen may also characterize another patient state. The preset rule can also define that the trend of change for heart rate and the trend of change for blood oxygen are in a same direction, then when both the trend of change for heart rate and the trend of change for blood oxygen are either an upward trend of change or a downward trend of change, the preset rule is satisfied.

After determining that the data that correspond to ECG parameter and Spo2 parameter satisfy one or more abnormal indicators included in the target rule, the processor determines one or more abnormal states of the patient that correspond to the target rule. For example, for the circulatory system, the patient state can be determined as an unstable circulatory state based on the target rule. When the patient state is not determined to be the unstable circulatory state, the circulatory system is assumed to be in a stable circulatory state. The patient state can also be determined as a cyclically stable state based on some target rules; for example, by determining whether a patient has arrhythmia, such as atrial fibrillation, and then experiences an abnormal trend of change, such as a decrease in blood pressure, which may due to a circulatory instability caused by atrial fibrillation.

For example, in the target rule that is associated with an abnormal state of a circulation state, one indicator is that "the heart rate has been continuously increasing in past four hours and blood oxygen has been continuously decreasing in past one hour". Furthermore, a time correlation may exist between trends of change for different patient data. The time correlation can include that respective occurrence time points of the trends of change for two types of patient data are at least partially the same, or a time difference between respective occurrence time points of the trends of change for two types of patient data does not exceed a preset time difference. For example, the upward trend of change for one type of patient data occurs within a few minutes of the downward trend of change for another type of patient data. The time correlation can also indicate that the trends of change for two types of patient data both occur within a larger time range, such as within 24 hours.

For an indicator that is associated with a waveform, a corresponding waveform of the patient data can be generated based on the patient data, and a second comparison result between the waveform of the patient data and a preset waveform template can be obtained. Based on the second comparison result, determine that whether the patient data satisfy one or more indicators in the target rule. For example, if an indicator is "a time interval between P wave and R wave of electrocardiogram waveform does not deviate from a corresponding time interval of the electrocardiogram waveform template by more than 100ms", then for this indicator, electrocardiogram data of the patient is obtained, an electrocardiogram waveform is generated based on the electrocardiogram data, and then the electrocardiogram waveform is compared with the electrocardiogram waveform template to determine whether the electrocardiogram data of the patient satisfy the indicator.

It can be understood that patient data of one type may only satisfy one indicator, or may satisfy multiple indicators simultaneously. Patient data of one type may only satisfy one target rule, or may satisfy multiple target rules simultaneously.

In addition to an abnormal state, when the patient data satisfy another preset rule in the rule library, it is also possible to conclude that the patient is in another state, such as a normal state, an unknown state, etc.

In some embodiments, patient data associated with different physiological structures can be analyzed separately to obtain abnormal states of different physiological structures. For example, after a user selects a physiological structure, the patient data that are associated with the physiological structure is analyzed to determine one or more target rules, which are satisfied by the patient data that are associated with the physiological structure. The following examples illustrate the patient date associated with each physiological structure.

A clinical parameter associated with the respiratory system includes but is not limited to an oxygenation index (PaO2/FiO2), a blood oxygen saturation (SpO2), a respiratory rate (RR), an inhaled oxygen concentration (FiO2), end-tidal carbon dioxide, a blood gas analysis parameter, and/or a ventilator parameter; wherein the blood gas analysis parameter includes lactate (Lac), an arterial partial pressure of oxygen (PaO2), an arterial partial pressure of carbon dioxide (PaCO2), etc., the ventilator parameter includes a tidal volume (Tv), a positive end expiratory pressure (PEEP), and a current mode of oxygen supply to the patient, such as using an SIMV ventilation mode, an intubation or a mask, etc. For example, if the target rule is that after using painkillers to relieve pain, etCO2 is too low for too long, RR is too low for too long, or SpO2 is too low for too long, then the respiratory system is correspondingly in an abnormal state, indicating that the painkillers may be excessive and inhibit the respiratory system, a dosage of the painkillers needs to be reduced. For example, by identifying that the patient has abnormalities, such as an upward trend of change for heart rate and an upward trend of change for respiratory rate, it is possible to determine that the abnormal state of the patient is dyspnea. For example, by identifying that the patient has abnormalities, such as an upward trend of change for respiratory rate, after the patient is disconnected from the ventilator, it is possible to determine that the abnormal state of the patient is a risk of deterioration caused by being disconnected from the ventilator.

A clinical parameter that is associated with the circulatory system includes but is not limited to: a shock index, a blood pressure, a cardiac output, lactate (Lac), a laboratory indicator and a hemodynamic parameter that are associated with hemodynamics and perfusion. Wherein, the blood pressure can be invasive or non-invasive, and the laboratory indicator includes but is not limited to hemoglobin (Hb or HGB), red blood cell count (RBC), pH, HCO3, and alkaline residue (BE); the hemodynamic parameter includes but is not limited to a central venous pressure (CVP), a peripheral vascular resistance index (SVRI), a pulmonary water index (ELWI), and a central venous oxygen saturation (ScvO2).

The clinical parameter that is associated with the nervous system includes but is not limited to a consciousness score, a brain blood pressure, a blood oxygen indicator, and a clinical assessment result that is associated with the nervous system. Wherein the commonly used consciousness score in clinical practice is Glasgow Coma Scale (GCS), but users are also allowed to define their own consciousness scoring rule. The clinical assessment result that is associated with the nervous system includes but is not limited to an assessment of pupil size, an assessment of pupil reflex, an assessment of limb muscle strength, etc.

The clinical parameter that is associated with the heart includes an assessment result for a heart-related risk, a heart rate, and a heart-related biochemical indicator. For example, by determining whether a patient has a trend of ST-segment elevation or depression, the patient may be determined as at a risk of myocardial ischemia. The assessment result for a heart-related risk can be, for example, a TIMI (Thrombolytic Therapy for Myocardial Infarction) score. If the patient has undergone GRACE (Global Acute Coronary Syndrome Registry) assessment, the assessment result for a heart-related risk can also include a GRACE score. The heart-related biochemical indicator can include creatine kinase isoenzyme (CK-MB), cardiac troponin (cTn), and NT proBNP.

The clinical parameter that is associated with the liver includes but is not limited to an assessment indicator for a liver function. A default assessment indicator for a liver function includes alanine aminotransferase (ALT), gamma glutamyl transferase (GGT), total bilirubin (Tbil), direct bilirubin (Dbil), and blood ammonia (AMM).

The clinical parameter that is associated with the kidneys includes but is not limited to a creatinine indicator, a fluid intake and a fluid output. The fluid intake includes a total 24-hour fluid intake and a fluid intake pumped into the body by an infusion pump within 24 hours. Furthermore, the liquid intake can also include a dietary liquid intake, etc. The liquid output includes 24-hour urine output, 24-hour drainage output, and a dehydration output of another device. The fluid output can also include sweating, excretion, vomiting, bleeding, etc.

The clinical parameter that is associated with infection includes a biochemical indicator that is associated with infection, and a vital sign parameter that is associated with infection. Wherein the biochemical parameter 503 that is associated with infection includes but is not limited to a white blood cell count (WBC), aC-reactive protein (CRP), procalcitonin (PCT), interleukin-6 (IL-6), and a neutrophil ratio (NEU%); the vital sign parameter associated with infection includes but is not limited to a body temperature.

The clinical parameter that is associated with coagulation includes but is not limited to an assessment result for a coagulation risk, a coagulation indicator, and a bleeding indicator. For a surgical patient, Caprini scale can be used for coagulation risk assessment, while for a non-surgical patient, Padua scale can be used for the coagulation risk assessment. The coagulation indicator 1202 includes at least one of: activated partial thromboplastin time (APTT), thrombin time (TT), fibrinogen (Fib), D-dimer, fibrin degradation product (FDP), or antithrombin III (AT-III). The bleeding indicator includes platelet (PLT), or occult blood (OB).

The clinical parameter that is associated with nutrition includes but is not limited to a monitoring result for energy metabolism, a supply of trace element, and a feeding method. Wherein, the monitoring result for energy metabolism includes an energy metabolism value (EE) and its trend of change. The trace element mainly includes calcium, iron, potassium, sodium, magnesium, etc. The feeding method mainly includes two categories: parenteral nutrition and enteral nutrition. Wherein, parenteral nutrition can be further subdivided into two types: deep vein-nutrition and superficial vein-nutrition. Intestinal nutrition can also be subdivided into nasal feeding, gastric duct feeding, etc.

When obtaining the abnormal state of the patient, it is possible to determine which type(s) of patient data satisfy one or more indicators. Based on the patient data that satisfy one or more indicators, corresponding waveform of the patient data can be generated, which waveform is associated with the abnormal state of the patient. For example, an indicator is "average heart rate greater than 90" and when the heart rate of the patient satisfies this indicator, a corresponding waveform of the heart rate is generated.

Step S300, display, on a display interface, state information that represents the abnormal state of the patient, and the waveform of the patient data, and provide graphic and text for explanatorily explaining an abnormal waveform segment in the waveform of the patient data; wherein the graphic and the text are associatively displayed with the abnormal waveform segment.

In some embodiments, at least one of the text and the graphic can also be used to display state information. When using text, a character string (i.e. text) that is associated with the abnormal state of the patient can be pre-configured, including a character string which represents an overall body of the patient, a physiological system of the patient, an organ of the patient, a physiological part of the patient or a tissue of the patient, as well as a character string which represents a designated abnormal state, such as circulatory system+possible shock/possible heart failure/possible internal bleeding, respiratory system+possible respiratory depression, nervous system+possible cerebral hemorrhage, urinary system+possible kidney failure, immune system+possible severe infection, etc. The character string can take various forms, as long as it can reflect the abnormal state of the patient, such as a directly displayed character string of "circulatory system is abnormal". Or a character string to represent heart failure can be "circulatory system may have heart failure", "patient may have heart failure", "patient is at risk of heart failure", etc. The character string can be pre-set by experts based on the abnormal state of each patient, or adjusted using natural language processing method to adapt to a designated abnormal state of a current patient. For example, a user can also be allowed to configure or modify the character string.

When a graphic is adopted, the graphic that is associated with the abnormal state of each patient can be stored in advance and can visually represent the abnormal state of the patient, and the graphic can be obtained for displaying after the abnormal state of the patient is determined. The graphic representing the abnormal state of the patient can correspond to an overall body of the patient, a physiological system of the patient, an organ of the patient, a physiological part of the patient or a tissue of the patient, and be marked with at least one of symbol information, color information, or text information located on or adjacent to the graphic (such as in a vicinity of the graphic) to present the abnormal state of the patient. For example, displaying the graphic that represents the circulatory system in red, so as to represent an abnormal state of the circulatory system.

The abnormal waveform segment in the waveform of the patient data include at least two meanings. One is that the patient data that correspond to the abnormal waveform segment itself are abnormal, for example, the corresponding patient data satisfy one or more indicators, or satisfy one or more target rules. Through the abnormal waveform segment, which patient data the abnormal state of the patient is caused by, as well as, specific values, trends of change, and other information of said patient data, can be reflected. Another meaning can be explained through a following scenario. First patient data and second patient data are two different types of data, but they have correlation in a clinical practice. When the first patient data satisfy one or more target rules (one or more indicators) in a first time period, the waveform of the patient data of the second patient data also has an abnormal waveform segment, and the abnormal waveform segment has a time correlation with the first time period. For example, a part of the waveform of the second patient data is partially abnormal in the second time period, and the time correlation can include that the first time period and the second time period are at least partially the same, or that a time difference between the first time period and the second time period does not exceed a preset time difference, For example, a few minutes after the first time period is referred to as the second time period, or both the first and second time periods can be within a same larger time range, such as within 24 hours. For example, in a clinical practice, heart rate and blood oxygen are often combined to determine a patient state. Heart rate and blood oxygen have the aforementioned correlation, with one being considered as the first patient data and the other as the second patient data.

It can be understood that abnormal waveform segment can be a waveform at a certain moment or within a certain time period. For example, when the patient data satisfy an indicator of "heart rate greater than 120", the abnormal waveform segment is a point on the waveform of the heart rate. When the patient data satisfy an indicator of "heart rate continuously increases for more than 4 hours", the abnormal waveform segment is a waveform segment on the waveform of heart rate, at which segment the heart rate continuously increases for more than 4 hours.

The graphic and the text that are used to provide explanatory explanation for abnormal waveform segment correspond to one or more target rules that are satisfied by the patient data, or in other words, correspond to one or more indicators that are satisfied by the patient data. Corresponding refers to that the graphic and the text are generated based on the target rule(s), and are also used to explain corresponding target rule(s) to a user. For example, the aforementioned text can be a description of one or more indicators in the target rule(s), such as directly displayed text of "average heart rate greater than 90".

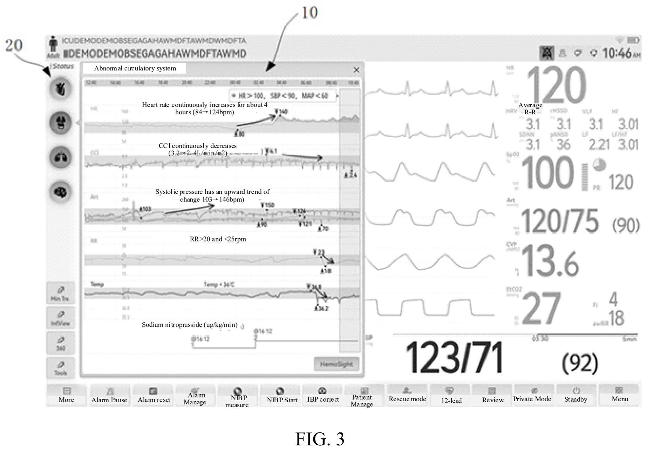

The text and the graphic, which correspond to a same target rule or a same indicator, are displayed adjacent to each other, that is, the text and the graphic, which correspond to a same indicator, are displayed adjacent to each other, as shown in FIG. 3. For example, if the patient data satisfy a first indicator and a second indicator, first text corresponding to the first indicator will be displayed adjacent to a first graphic corresponding to the first indicator, and second text corresponding to the second indicator will be displayed adjacent to a second graphic corresponding to the second indicator, wherein the first indicator and the second indicator are the two indicators satisfied by the patient data. For example, the first indicator is "heart rate continuously increases for more than 4 hours", and the second indicator is "myocardial contractility index continuously decreases". The patient data satisfy both the first indicator and the second indicator. In FIG. 3, the first text includes "HR continuously increases for about 4 hours", the first graphic includes an arrow representing an upward trend of change for heart rate, the second text includes "CCI continuously decreases", and the second graphic includes an arrow representing a downward trend of change for CCI. Compared to the second graphic, the first text is closer to the first graphic, and compared to the first graphic, the second text is closer to the second graphic, allowing a user to easily understand the relationship between the graphic and the text without confusion.

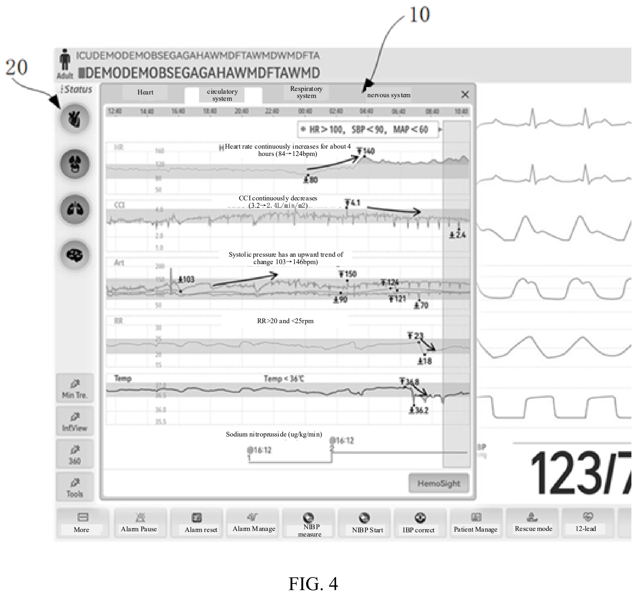

In the previous description, it was explained that the indicator in the target rule includes but is not limited to an indicator that is associated with a threshold, an indicator that is associated with a trend of change, an indicator that is associated with a waveform shape, etc. When the patient data satisfy different types of indicators, display manners of graphic and text to explain the abnormal waveform segment can also vary. Further explanation will be provided below in conjunction with FIGS. 3 and 4.

The waveforms of the patient data in FIGS. 3 and 4 are trend charts over a time period, for example, the waveform of heart rate is a trend chart of heart rate. The patient data satisfy at least two target rules. The first target rule includes three indicators that are associated with a threshold, namely "HR greater than 100", "SBP less than 90", and "MAP less than 60". The second target rule includes five indicators that are associated with a trend of change, namely "heart rate continuously increases for more than 4 hours", "myocardial contractility index continuously decreases for more than 4 hours", "systolic blood pressure continuously increases for more than 4 hours", "respiratory rate continuously decreases for more than 1 hour", and "body temperature continuously decreases for more than 1 hour". In the following description, only examples including two target rules mentioned above are included. In FIG. 3, other target rules (indicators), such as "respiratory rate greater than 20 and less than 25" are also included.

In FIG. 3, the first target rule is explained directly with text "HR>100, SBP<90, MAP<60". At the same time, the text points to a color block, and the patient data within the time period represented by the color block satisfy "HR>100, SBP<90, MAP<60". The waveform of each patient data marked by the color block is an abnormal waveform segment corresponding to the first target rule. It can be seen that in addition to the waveform of heart rate, other waveforms of the patient data during this time period are also marked. User can view a respiratory rate, a systolic blood pressure, etc., during this time period, so as to further determine the abnormal state of the patient based on the first target rule. Especially, an abnormal waveform segment on a waveform of infusion data is marked, and user can observe changes in the infusion data before and after the abnormal segment to assess an infusion effect. In addition to the color block, the abnormal waveform segment can also be marked by changing its color, line type, and so on. In other embodiments, only the abnormal waveform segment corresponding to the first target rule on the waveform of heart rate can be marked.

In FIG. 3, the trend of change for the patient data is also marked adjacent to the abnormal waveform segment corresponding to the second target rule on the waveform of the patient data. Draw a trend chart of patient data and present types (upward, downward, fluctuated, abrupt) and durations of trend of change in the physiological parameters. Then, present time and magnitude of an abnormality of a physiological parameter through an arrow, wherein a presenting manner includes but is not limited to: using a start point of a trend of change for the physiological parameter as a start point of the arrow, and using an end point of the trend of change for the physiological parameter as an end point of the arrow; using a start point of the trend of change for the physiological parameter as a start point of the arrow, and using time and amplitude of an end point of the trend of change for the physiological parameter as an end point of the arrow (i.e. the arrow is drawn vertically and upwardly). In addition, the abnormal segment of the physiological parameter can also be highlighted. Continuing with heart rate as an example, the text "HR continuously increases for about 4 hours (84->124bpm)" is displayed adjacent to the abnormal waveform segment corresponding to the second target rule, and an arrow indicating the upward trend of change for the heart rate is displayed above the abnormal waveform segment. In some embodiments, the heart rate before the change can also be displayed at or adjacent to the start position of the abnormal waveform segment, and the heart rate after the change can be displayed at or adjacent to the end position of the abnormal waveform segment. In the current FIG. 3, the heart rate is marked on the electrocardiogram waveform in a form of dot.

From FIGS. 3 and 4, one type of waveform of the patient data can have more than one abnormal waveform segment. The electrocardiogram waveform in FIG. 3 includes at least three abnormal waveform segments, from left to right: an abnormal waveform segment that corresponds to a second target rule and represents an upward trend of change for heart rate; an abnormal waveform segment with a heart rate that satisfies an indicator of "HR greater than 100", wherein a color below said abnormal waveform segment is different from that of other positions in the waveform of the heart rate in the figure; an abnormal waveform segment that corresponds to a first target rule.

It should also be noted that an abnormal state of the circulatory system is obtained in FIGS. 3 and 4. The patient data that are associated with the abnormal state of the circulatory system may not only include those shown in FIGS. 3 and 4, but also include other patient data. Due to a size of the display interface, the waveform of the patient data that has a greater impact on the circulatory system is displayed.

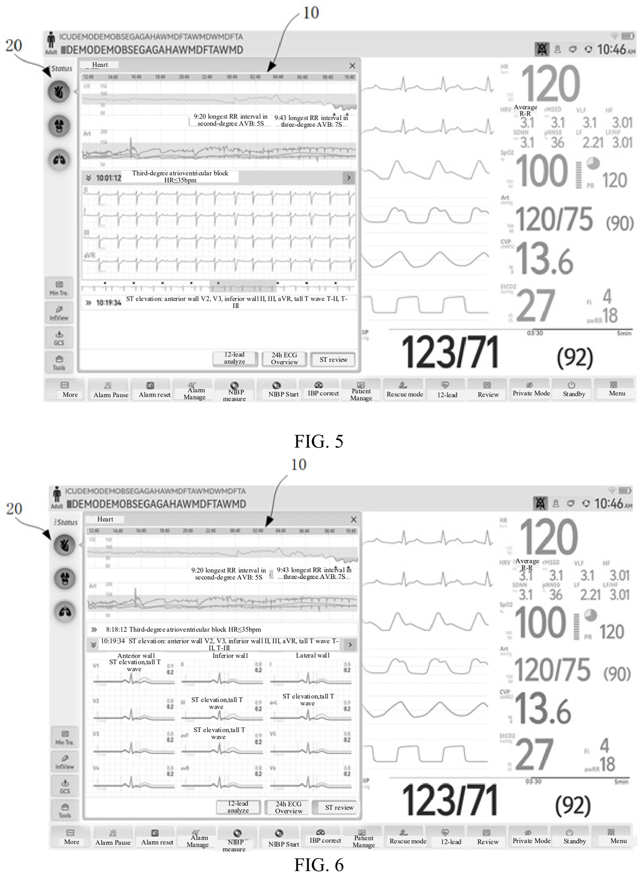

Continuing to refer to FIGS. 5 and 6, the patient data in FIGS. 5 and 6 satisfy at least two target rules. To distinguish said at least two target rules from the target rule mentioned above, said two target rules are defined as a third target rule and a fourth target rule. The third target rule includes "third-degree atrioventricular block, heart rate less than or equal to 35", the fourth target rule includes "ST elevation, tall T-wave". In addition to the text corresponding to the target rule, the abnormal waveform segment corresponding to the third target rule is marked with a dark background in FIG. 5. In FIG. 6, the abnormal waveform segment corresponding to the fourth target rule is compared and displayed with a preset waveform template to help a user to further grasp a specific situation of the abnormal state.

From the above, it can be seen that a technical solution of this disclosure is not a general or an arbitrary combination of waveform(s), text, and graphic, instead, through directional text and graphic to provide explanatory explanation for the abnormal waveform segment, a user can more intuitively understand how the abnormal state of the patient is obtained, based on which patient data and which situation the abnormal state of the patient is obtained, so as to facilitate targeted diagnosis and treatment for the patient, such as timely symptomatic treatment.

In some embodiments, a state analysis window 10 can be specifically arranged to display the state information that represents the abnormal state of the patient, the waveform of the patient data, and the graphic and the text to explain the abnormal waveform segment mentioned earlier. This state analysis window 10 can be understood as a separate and independent window from a common region or a window for displaying patient data, alarm data, etc., on a medical device, such as a monitor.

On the display interface of the embodiment of this disclosure, in addition to displaying the state analysis window 10, another content, such as a real-time physiological parameter, a real-time waveform, etc., is also displayed, wherein said another content is defined as "other content" in this disclosure. The aforementioned state analysis window 10 can partially block an original content when the state analysis window 10 is displayed. Alternatively, the state analysis window 10 can also completely coexist with said another content originally presented on the display interface, depending on whether the state analysis window 10 is displayed for a long term or as a pop-up, as well as a size of the display, a number of parameter(s) and/or content originally displayed, and so on. Below, we will illustrate with reference to FIG. 3.

In addition to displaying the state analysis window 10 mentioned earlier, the display interface in FIG. 3 also shows a real-time waveform and a values of the patient data, as well as some interface controls, such as interface controls for alarm and reset.

In some embodiments, the state analysis window 10 is displayed on the display interface for a long term, such as on a left side of the figure. During a long-term display, the state analysis window 10 will not completely block other content on the display interface.

In some embodiments, the state analysis window 10 is only automatically displayed on the display interface, when the abnormal state of the patient is obtained, for example, in a pop-up manner. When abnormal states of two or more physiological structures are obtained in the aforementioned physiological structures, priority can be given to display state information, waveform of the patient data, and the aforementioned text and graphic, which are associated with a more severe physiological structure. For example, in FIG. 3, assuming that respective abnormal states of the circulatory system and the nervous system are obtained based on analyzing the patient data, and a degree of abnormality of the circulatory system exceeds that of the nervous system, when the state analysis window 10 is automatically displayed, the state analysis window 10 displays the state information of the circulatory system, the waveform of the patient data associated with the circulatory system, as well as the graphic and the text to explain the abnormal waveform segment of the circulatory system.

In some embodiments, when obtaining the abnormal state of the patient, the display interface prompts the user to input an operation to open the state analysis window 10, and displays the state analysis window 10 after detecting the operation to open the state analysis window 10. That is to say, when receiving an abnormal patient state, prompt the user to open the state analysis window 10. An exemplary prompt method is to display the state analysis window 10 in a form of a pop-up, when a designated icon 20 in FIG. 3 is triggered.

Wherein, the designated icon 20 can exist continuously or only exist when the abnormal state of the patient is obtained. For example, the designated icon 20 can be displayed at a fixed position on the display interface for a long term, and when an abnormal state of the patient is detected, a display mode of the designated icon 20 can be changed (such as flashing, enlarging, color highlighting, etc.) to prompt the user to trigger the designated icon 20 to open the state analysis window 10. Alternatively, when an abnormal state of the patient is detected, a designated icon 20 automatically appears on the display interface to prompt the user to trigger the designated icon 20 to open the state analysis window 10. Designated icons 20 in FIG. 3 are also in a one-to-one correspondence with four physiological systems. When an abnormal state of one of the physiological systems is obtained, a display mode of a corresponding designated icon 20 can be changed to prompt the user to trigger the corresponding designated icon 20. When the corresponding designated icon 20 is triggered, the state information of the physiological system corresponding to the designated icon 20, the associated waveform of the patient data, and the graphic and the text to explain the abnormal waveform segment will be displayed in the state analysis window 10. For example, in FIG. 3, a designated icon 20 corresponding to the circulatory system is triggered.

In addition, no matter in a long-term display, a pop-up display for abnormity, or a pop-up display after triggering a designated icon 20, in embodiments of this disclosure, a position of the state analysis window 10 on the display interface is user adjustable. For example, a user can preset the position of the state analysis window 10 on the display interface, or after the state analysis window 10 is displayed, a user can change the position of state analysis window 10 at any time (such as by dragging).

Based on the different display methods of the state analysis window 10 mentioned above, another content can be displayed differently. In one example, the display interface also displays the another content different from the state analysis window 10. When the state analysis window 10 is displayed on the display interface for a long term, the state analysis window 10 and another content are displayed independently and completely in different regions of the display interface. When the state analysis window 10 is automatically displayed, or displayed in the form of a pop-up window after being triggered through a designated icon 20 by a user, the state analysis window 10 blocks at least a part of the another content, or a layout and/or a size of the another content is adaptively adjusted to be completely displayed on the display interface.

In some embodiments, as shown in FIG. 4, the state analysis window 10 is also used to display a tag that is in a one-to-one correspondence with at least one physiological structure. When any one tag is triggered, a waveform of the patient data, graphic and text, which are associated with the abnormal state of a corresponding physiological structure, are displayed in the state analysis window 10. For example, if the waveform of the patient data, the graphic and the text, which are associated with the abnormal state of the circulatory system, are displayed now, and the user triggers the nervous system, the state analysis window 10 can display the waveform of the patient data, graphic and text, which are associated with the abnormal state of the nervous system.

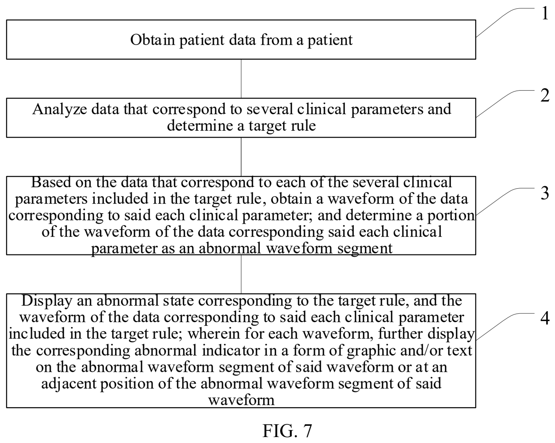

Based on the system for processing vital information and its method for processing vital information, this disclosure also provides a method for processing vital information, as shown in FIG. 7, which may include the following steps.

Step 1, the processor 120 obtains patient data of a patient, wherein the patient data includes data corresponding to multiple clinical parameters, which data are associated with a physiological structure and obtained (such as collected) within a preset time range. The preset time range can be preset by a user, such as 24 hours mentioned in the previous embodiment. Taking 24 hours as an example, it is equivalent to that the processor 120 obtains data of various clinical parameters that are associated with various physiological structures within 24 hours (such as values of clinical parameters at different times within 24 hours).

The obtained patient data can be patient data associated with one physiological structure or multiple physiological structures. This embodiment takes the latter as an example to illustrate, so that through this method for processing vital information, it is possible to determine whether the multiple physiological structures have abnormal state(s).

Clinical parameters that are pre-associated with each physiological structure are different and have their own emphasis. For example, the physiological structure includes at least a physiological system, wherein the physiological system includes at least one of: a nervous system, a circulatory system, and a respiratory system. When the physiological system includes the nervous system, a plurality of clinical parameters that are associated with the nervous system are a plurality of clinical parameters that are associated with a brain nerve. Specifically, the plurality of clinical parameters that are associated with the nervous system include but are not limited to a consciousness score, a brain blood pressure, a blood oxygen indicator, and a clinical assessment result that is associated with the nervous system. When the physiological system includes a circulatory system, a plurality of clinical parameters that are associated with the circulatory system are a plurality of clinical parameters that are associated with hemodynamics or perfusion. Specifically, the plurality of clinical parameters that are associated with the circulatory system may include but are not limited to a shock index, a blood pressure, a cardiac output, lactate (Lac), a laboratory indicator that is associated with hemodynamics and perfusion, and a hemodynamic parameter. When the physiological system includes a respiratory system, a plurality of clinical parameters that are associated with the respiratory system are a plurality of clinical indicators that are associated with oxygenation. Specifically, the plurality of clinical parameters that are associated with the respiratory system may include but are not limited to an oxygenation index (PaO2/FiO2), a blood oxygen saturation (SpO2), a respiratory rate (RR), an inhaled oxygen concentration (FiO2), end tidal carbon dioxide, a blood gas analysis parameter, and/or a ventilator parameter, wherein the blood gas analysis parameter includes lactate (Lac), an arterial oxygen partial pressure (PaO2), an arterial carbon dioxide partial pressure (PaCO2), etc. The specific process of obtaining patient data by the processor 120 is as described in step S100 of the previous embodiment, and will not be repeated here.

Each physiological structure is pre-associated with one or more rules, and each rule of the physiological structure is pre-associated with an abnormal state of the physiological structure. In other words, each rule of the physiological structure is configured to determine an abnormal state of the physiological structure, and each rule is equivalent to a determination condition for one abnormal state, and is composed of multiple clinical parameters satisfying abnormal indicator(s). Rules associated with different physiological structures are different, and rules associated with different abnormal states of a same physiological structure are also different. Each rule includes abnormal indicator(s) that corresponds(correspond) to multiple clinical parameters, wherein the abnormal indicator(s) is(are) "indicator(s)" mentioned in the previous embodiment. An abnormal indicator is equivalent to one of multiple sub-rules that are required to determine an abnormal state. If multiple sub-rules are satisfied simultaneously, it indicates that a determination condition for the abnormal state is satisfied, and thus the physiological structure can be determined to have this abnormal state. From this, it can be seen that the abnormal state of this disclosure is different from conventional parameter abnormalities, such as a physiological parameter exceeding a normal range, but rather reflects an abnormality in a physiological structure. The causes of such abnormal state are more complex, and it is more difficult for a doctor to determine and handle. In this disclosure, the causes of such abnormal state can be determined by the processor 120, with a high degree of automation. The abnormal indicator of the clinical parameter may be a routine abnormality, such as a clinical parameter exceeding a normal range, or not a routine abnormality, such as a clinical parameter exceeding a certain range but still within the normal range. However, when this situation occurs simultaneously or before or after another abnormal indicator of another clinical parameter, it indicates that a physiological structure of the patient is in an abnormal state. Therefore, the clinical parameter(s) in this situation also need to be paid attention to.

Step 2, the processor 120 analyzes data that correspond to several of the multiple clinical parameters to determine a target rule. Wherein, the target rule includes the several clinical parameters analyzed, wherein data, which correspond to each of the several clinical parameters that is included in the target rule, have reached an abnormal indicator that corresponds to said each clinical parameter. In other words, when data, which correspond to all clinical parameters included in a rule mentioned in step 1, satisfy abnormal indicator(s) that corresponds(correspond) to their respective clinical parameters, said rule is a target rule. If the processor 120 is unable to determine the target rule, it indicates that the physiological structure of the patient is not in an abnormal state.

There are various ways to determine the target rule, and three of them are listed below for detailed explanation.

The first method is to first determine whether data of all clinical parameter satisfy the abnormal indicators, and then determine the target rule. For example, the processor 120 determines whether the data that correspond to each clinical parameter satisfy the abnormal indicator corresponding to said clinical parameter.

There can be one or more types of abnormal indicators corresponding to the clinical parameters included in each rule. For the system for processing vital information, there can be three types of abnormal indicators. One type of abnormal indicator is an abnormal indicator that is associated with a trend of change. In this embodiment, at least one abnormal indicator corresponding to at least one of the several clinical parameters included in the target rule is associated with the trend of change for the at least one clinical parameter, that is, the target rule includes at least one abnormal indicator that is associated with the trend of change. For ease of differentiation, the clinical parameter corresponding to this type of abnormal indicator can be referred to as a first clinical parameter. For such abnormal indicator, a designated duration of the trend of change for the first clinical parameter may not be less than a preset duration associated with the first clinical parameter in advance. The processor 120 specifically determines whether the data of the first clinical parameter satisfy the abnormal indicator corresponding to the first clinical parameter as follows. The processor 120 obtains a waveform based on the data of the first clinical parameter, identifies a waveform segment that conform to a trend of change in the waveform, and then determines whether a duration of the waveform segment is not shorter than a preset duration; if yes, determines that the waveform segment is an abnormal waveform segment and the abnormal waveform segment satisfies (or reaches) the abnormal indicator. Of course, the processor 120 can also use other methods to determine whether the clinical parameter data satisfy the abnormal indicator, such as using pre-trained machine/deep learning model to decide.

Another type of abnormal indicator is an abnormal indicator that is associated with a threshold. In some embodiments, at least one of the several clinical parameters included in the target rule corresponds to an abnormal indicator that is associated with a threshold of said at least one clinical parameter. For ease of differentiation, the clinical parameter corresponding to this type of abnormal indicator can be referred to as a second clinical parameter. For such abnormal indicator, it can specifically be that the value of the second clinical parameter exceeds a preset value. The processor 120 specifically determines whether the data of the second clinical parameter satisfy the abnormal indicator corresponding to the second clinical parameter as follows. The processor 120 determines whether a value of the second clinical parameter data exceeds a preset value, if there is a value that exceeds the preset value, determines that the value has reached the abnormal indicator, and determines a waveform segment that corresponds to the value or before and after the value is the abnormal waveform segment.

Another type of abnormal indicator is an abnormal indicator that is associated with a waveform morphology. In some embodiments, at least one abnormal indicator corresponding to at least one of the several clinical parameters included in the target rule is associated with a waveform morphology of the clinical parameter. For ease of differentiation, the clinical parameter corresponding to such abnormal indicator can be referred to as a third clinical parameter. For such abnormal indicator, a designated deviation between a waveform of the data of the third clinical parameter and a preset waveform template should not exceed or should exceed a preset deviation. The processor 120 specifically determines whether the data of the third clinical parameter satisfy the abnormal indicator corresponding to the third clinical parameter as follows. The processor 120 obtains a waveform based on the data of the third clinical parameter; segments the waveform to obtain multiple waveform segments, compares respective deviations between the multiple waveform segments and a preset normal waveform template with a preset deviation, if a deviation exceeds the preset deviation, determines a corresponding waveform segment to be the abnormal waveform segment. Alternatively, the processor 120 can also compare respective deviations between the multiple waveform segments and a preset abnormal waveform template, if a deviation does not exceed a preset deviation, determine a corresponding waveform segment to be the abnormal waveform segment. The abnormal waveform segment indicates that the abnormal indicator has been reached. Of course, the processor 120 can also use other methods to determine whether the data of the third clinical parameter satisfy its abnormal indicator, such as using pre-trained machine/deep learning model to decide.

From the above content, it can be seen that clinical parameters correspond to abnormal indicators, and clinical parameter data that reach the abnormal indicators correspond to abnormal waveform segments.

Furthermore, the processor 120 determines the target rule based on each abnormal indicator reached, for example, matches each abnormal indicator reached in a preset rule library, and the matched rule is the target rule. Since each rule is associated with multiple abnormal indicators of multiple clinical parameters, as long as data of all clinical parameter included in one rule satisfy their abnormal indicators, the rule is determined as the target rule.

The second method is to determine whether data of all clinical parameter included by a rule satisfy their corresponding abnormal indicators, in units of rule. For example, for each rule, the processor 120 respectively determines whether data of all clinical parameters that correspond to said rule satisfy the abnormal indicators corresponding to said all clinical parameters; if yes, determine said rule as the target rule. To determine whether corresponding data of a clinical parameter satisfy an abnormal indicator, please refer to the previous content, which will not be elaborated here.

The third method is to determine whether data of all clinical parameters of all rules that are included by a physiological structure satisfy their corresponding abnormal indicators, in units of physiological structure. For example, the processor 120 analyzes data that correspond to clinical parameters included in all rules associated with each physiological structure to determine the target rule. Wherein, the specific methods for analyzing the data that correspond to clinical parameters to determine the target rules are described in the first and second methods mentioned above, which will not be elaborated here.

For a rule that is pre-associated with the circulatory system, its pre-associated abnormal state can be shock. This rule includes an abnormal indicator associated with a trend of change for a PI parameter or a CQI parameter, as well as an abnormal indicator associated with a trend of change for a blood pressure. Specifically, the abnormal indicator associated with a trend of change for a PI parameter or a trend of change for a CQI parameter can be that perfusion reflected by the PI parameter or the CQI parameter show a downward trend; the abnormal indicator associated with a trend of change for a blood pressure can be that the trend of change for the blood pressure shows a downward trend.

For another rule that is pre-associated with the circulatory system, its pre-associated abnormal state can be a circulatory deterioration. This rule includes an abnormal indicator associated with a trend of change for a heart rate, and an abnormal indicator associated with a trend of change for blood oxygen. Specifically, the abnormal indicator associated with the trend of change for the heart rate can be that the trend of change for the heart rate is an upward trend; the abnormal indicator associated with a trend of change for blood oxygen can be that the trend of change for the blood oxygen is a downward trend. The specific details have been elaborated in the aforementioned embodiments and will not be repeated here.

For another rule that is pre-associated with the circulatory system, its pre-associated abnormal state can be a circulatory instability. This rule includes an abnormal indicator of an electrocardiogram (ECG) signal and an abnormal indicator associated with a trend of change for blood oxygen. The abnormal indicator of an electrocardiogram (ECG) signal can be atrial fibrillation reflected by the ECG signal; the abnormal indicator associated with a trend of change for blood oxygen can be that the trend of change for blood oxygen is a downward trend.

For another rule that is pre-associated with the respiratory system, its pre-associated abnormal state can be respiratory distress. This rule includes an abnormal indicator associated with a trend of change for a heart rate and an abnormal indicator associated with a trend of change for a respiratory rate. The abnormal indicator associated with a trend of change for a heart rate can specifically be that the trend of change for the heart rate is an upward trend; the abnormal indicator associated with a trend of change for a respiratory rate can specifically be that the trend of change for the respiratory rate is an upward trend.

The processor 120 determines whether data of relevant clinical parameters satisfy the abnormal indicators included in the rules of the physiological structure listed here. Please refer to the relevant content of the previous embodiments for details, which will not be repeated here.

Step 3, the processor 120 obtains a waveform of the data that corresponding each of the several clinical parameters that is included in the target rule; determines a portion of the waveform of the data corresponding said each clinical parameter as an abnormal waveform segment, wherein the portion of the waveform of the data corresponding to said each clinical parameter reaches the abnormal indicator corresponding to said each clinical parameter. The waveform is mainly obtained for subsequent display.

For the clinical parameter (first clinical parameter), which corresponds to the abnormal indicator that is associated with the trend of change, a duration of the abnormal waveform segment of the corresponding waveform of said parameter is not less than a preset duration that is pre-associated with said clinical parameter. Moreover, the trend of change for the abnormal waveform segment includes an upward trend of change in general, a downward trend of change in general, a fluctuated trend of change in general, or an abrupt trend of change in general, within the duration of the abnormal waveform segment. That is to say, the trend of change for the clinical parameter or the abnormal waveform segment can be classified into four types. Specifically, an upward trend of change in general within the duration of the abnormal waveform segment, means that within the duration of the abnormal waveform segment, there is an upward trend of change in general and a fluctuation is allowed during this period. In other words, within the duration of the abnormal waveform segment, a linear regression slope of the abnormal waveform segment is greater than zero, and a difference between the abnormal waveform segment and a linear regression line is within a preset range (fluctuations are allowed). The processor 120 can determine whether the data of the first clinical parameter satisfy the abnormal indicator (whether there is an abnormal waveform segment) based on this condition. Similarly, within the duration of the abnormal waveform segment, a downward trend of change in general within the duration of the abnormal waveform segment, means that within the duration of the abnormal waveform segment, there is a downward trend of change in general and a fluctuation is allowed during this period. In other words, within the duration of the abnormal waveform segment, a linear regression slope of the abnormal waveform segment is less than zero, and a difference between the abnormal waveform segment and a linear regression line is within a preset range. The processor 120 can determine whether the data of the first clinical parameter satisfy the abnormal indicator (whether there is an abnormal waveform segment) based on this condition. Similarly, a fluctuated trend of change in general within the duration of the abnormal waveform segment, means that the overall duration of the abnormal waveform segment shows no upward or downward trend, but have fluctuations allowed during this period. In other words, there is no upward trend or downward trend in the duration of the abnormal waveform segment, and numerical values increase and decrease repeatedly, with a fluctuation amplitude exceeding a preset amplitude. The processor 120 can determine whether the data of the first clinical parameter satisfy the abnormal indicator based on this condition. Specifically, an abrupt trend of change in general within the duration of the abnormal waveform segment, means that there are two values within the duration of the abnormal waveform segment which values have a difference value that is greater than a preset amplitude (larger than the aforementioned fluctuation amplitude), that is, the values of the clinical parameter change dramatically in a short time period. The processor 120 can determine whether the data of the first clinical parameter satisfy the abnormal indicators based on this condition.

The order of steps 3 and 2 is not limited, and they can be performed simultaneously or sequentially. For example, if an abnormal waveform segment is used to determine whether the date of the clinical parameter satisfy an abnormal indicator in step 2, step 3 can be executed first and then step 2. If not, step 2 can be executed first and then step 3.

Step 4, the processor 120 controls a display interface of a display to display the abnormal state of the physiological structure that corresponds to the target rule, and to display the waveform of the data corresponding to said each clinical parameter that is comprised in the target rule (as shown in each waveform in the left state analysis window 10 of FIGS. 3 and 4). As shown in FIGS. 3 and 4, a designated icon 20 is an abnormal state of a physiological structure, and there are various ways to display the abnormal state, such as displaying its corresponding designated icon 20, directly displaying the characters of the abnormal state (such as text, code, etc.), and of course, it can also be in other forms, as long as it allows doctors to see the screen and know what kind of abnormal state the patient is currently experiencing. At the same time, the processor 120 displays respective abnormal indicators corresponding to each waveform (corresponding clinical parameters) in the form of graphic and/or text on respective abnormal waveform segments or at adjacent positions of said respective abnormal waveform segments for each waveform. In the existing technology, the determination of abnormal states of physiological structures is often complex. Usually, the system uses models to automatically determine and provide results. The determination of AI model is in a black box mode, and an accuracy of its determination cannot be known. Moreover, as long as the determination is automatically given by the system, doctors usually doubt its credibility. This disclosure not only improves the accuracy of abnormal state determination through rule-based determination, but more importantly, displays the waveforms of various clinical parameters used to determine the abnormal states. Moreover, the corresponding abnormal indicators are displayed in the form of graphic and/or text on the abnormal waveform segments or at adjacent positions of the abnormal waveform segments. This not only prompts the doctor where the abnormal waveform segment is located, but also indicates the possible situations that may cause abnormal states in this clinical parameter (abnormal indicators). Doctors can quickly see this information and obtain the reliability (accuracy) of the abnormal state automatically given by the system based on this information, which improves their work efficiency.

The adjacent positions of the abnormal waveform segments can include: a position located above and adjacent to the abnormal waveform segment, or a position located below and adjacent to the abnormal waveform segment. A adjacent or neighboring position can be a position closer to the abnormal waveform segment than to the normal waveform segment.

The processor 120 can also differentiate abnormal waveform segments in the waveform, such as highlighting them, displaying them in different colors, etc., to highlight the abnormal waveform segments.

As shown in FIGS. 3 and 4, for a clinical parameter (first clinical parameter) corresponding to the abnormal indicator associated with the trend of change, a start position and/or an end position of the abnormal waveform segment corresponding to the clinical parameter are marked on the abnormal waveform segment of the waveform, and value(s) of the clinical parameter at the start position and/or the end position is(are) also displayed. For example, the values of the clinical parameter are respectively displayed adjacent to the start position and adjacent to the end position. This can also indicate to the doctor the location and duration of abnormal waveform segment, as well as a magnitude of change in the abnormal waveform segments.

Graphic can include an arrow. For a clinical parameter corresponding to an abnormal indicator associated with a trend of change, the processor 120 can display an arrow indicating the trend of change for the abnormal waveform segment and explanatory text for the abnormal indicator corresponding to the clinical parameter on or adjacent to the abnormal waveform segment of the waveform corresponding to the clinical parameter. By using an arrow to indicate the trend of change for the abnormal waveform segment, doctor can easily see at a glance and the human-machine interaction efficiency is high.

Wherein, the arrow can take the start position of the abnormal waveform segment as a start point of the arrow, and take the end position as an end point of the arrow. In some embodiments, the start position of the abnormal waveform segment can also be used as the start point of the arrow, and the time of the end position of the abnormal waveform segment plus the amplitude of the end position can be used as the ending point of the arrow (i.e., the arrow is drawn upward or downward). The direction or orientation indicated by the arrow corresponds to the trend of change (upward or downward). The description related to arrow and text is described in the previous embodiments and will not be elaborated here.

In one embodiment, when the abnormal state of the physiological structure (such as the heart) corresponding to the target rule includes RonT (R-on-T phenomenon) arrhythmia or QTc (e.g. QTc=QT+1.75 (HR-60)) prolongation, the processor 120 also obtains dosage data infused to the patient within a preset time range, and displays the waveform of the dosage data on the display interface, as shown in bottom box 10 of FIGS. 3 and 4. This type of abnormal state may be a risk caused by a side effect of drug. If the doctor determines that a blood pressure of the patient tends to decrease after reducing a dose of vasopressors, then this abnormal state may be a risk caused by the side effect.

The various waveforms mentioned above can be aligned in time, as shown in FIGS. 3 and 4. The waveforms share a common timeline and are arranged vertically between each other, making it easier for doctors to compare the trends of change for various clinical parameters before and after a certain moment, and thus understand the correlation between changes in various clinical parameters.

Technicians in this field can understand that all or part of functions of various methods in the above embodiments can be implemented through hardware or computer programs. When all or part of functions in the above embodiments are implemented through a computer program, the program can be stored in a computer-readable storage medium, which can include read-only memory, random access memory, magnetic disk, optical disk, hard disk, etc. The program can be executed by a computer to achieve the above functions. For example, store the program in a device memory, and when execute the program by a processor, all or part of the above functions can be achieved. In addition, when all or part of functions in the above embodiments are implemented through a computer program, the program can also be stored on a storage media, such as server, another computer, disk, CDs, flash drive, or portable hard drive. They can be downloaded or copied to a memory of a local device, or a system of a local device can be updated in version. When the program in the memory is executed by a processor, all or part of the functions in the above embodiments can be implemented.

The above application of specific embodiment to illustrate this disclosure is only intended to assist in understanding this disclosure and is not intended to limit this disclosure. For general technical personnel in this field, changes can be made to the specific implementation methods based on the idea of this disclosure.

## Claims

1. A method for processing vital information that is applicable to a system for processing vital information, **characterized in that**, the system for processing vital information comprises a memory, a processor and a display, wherein the memory is configured to store an executable program, the processor is configured to execute the executable program, so as to enable the processor to implement following operations:
obtaining patient data from a patient, wherein the patient data comprises data corresponding to multiple clinical parameters, which data are associated with a physiological structure and obtained within a preset time range; wherein the physiological structure is pre-associated with one or more rules, each rule is pre-associated with one abnormal state of the physiological structure, wherein each rule comprises: multiple abnormal indicators that correspond to the multiple clinical parameters; the physiological structure at least comprises a physiological system, wherein the physiological system comprises at least one of: a nervous system, a circulatory system or a respiratory system;
analyzing data that correspond to several of the multiple clinical parameters and determining a target rule; wherein the target rule comprises the several clinical parameters analyzed; wherein data, which correspond to each of the several clinical parameters that is comprised in the target rule, have reached an abnormal indicator that corresponds to said each clinical parameter; wherein an abnormal indicator, which corresponds to at least one of the several clinical parameters analyzed that is comprised in the target rule, is at least associated with a trend of change for said at least one clinical parameter;
based on the data that correspond to each of the several clinical parameters that is comprised in the target rule, obtaining a waveform of the data corresponding to said each clinical parameter; and determining a portion of the waveform of the data corresponding said each clinical parameter as an abnormal waveform segment, wherein the portion of the waveform of the data corresponding to said each clinical parameter reaches the abnormal indicator corresponding to said each clinical parameter; and
controlling a display interface of the display to display the abnormal state of the physiological structure that corresponds to the target rule, and to display the waveform of the data corresponding to said each clinical parameter that is comprised in the target rule; wherein for each waveform, further displaying the corresponding abnormal indicator in a form of graphic and/or text on the abnormal waveform segment of said waveform or at an adjacent position of the abnormal waveform segment of said waveform.

2. The method according to claim 1, **characterized in that**, the graphic comprises an arrow;
for each waveform, further displaying the corresponding abnormal indicator in a form of graphic and/or text on the abnormal waveform segment of said waveform or at an adjacent position of the abnormal waveform segment of said waveform, comprises:
for a clinical parameter, which corresponds to the abnormal indicator that is associated with the trend of change; displaying an arrow and text on the abnormal waveform segment of the waveform corresponding to said clinical parameter or at an adjacent position of the abnormal waveform segment of the waveform corresponding to said clinical parameter, wherein the arrow represents the trend of change of the abnormal waveform segment, and the text explains the abnormal indicator that corresponds to said clinical parameter.

3. The method according to claim 2, **characterized in that**, a duration for the abnormal waveform segment of the waveform corresponding to said clinical parameter, is not shorter than a preset duration that is associated with said clinical parameter;
the trend of change of the abnormal waveform segment comprises an upward trend of change in general, a downward trend of change in general, a fluctuated trend of change in general, or an abrupt trend of change in general, within the duration for the abnormal waveform segment.

4. The method according to claim 2, **characterized in that**, further comprising:
for said clinical parameter, which corresponds to the abnormal indicator that is associated with the trend of change; marking, on the abnormal waveform segment of the waveform corresponding to said clinical parameter, a start position and/or an end position of the abnormal waveform segment, and displaying values/value of said clinical parameter at the start position and/or the end position.

5. The method according to claim 1, **characterized in that**, analyzing data that correspond to several of the multiple clinical parameters and determining a target rule, comprise:
for the data, which correspond to said each clinical parameter: determining whether said data satisfy the abnormal indicator that corresponds to said each clinical parameter, and determining the target rule based on the abnormal indicator that is satisfied;
for each rule: determining whether data, which correspond to all clinical parameters that are comprised in said rule, satisfy abnormal indicators that correspond to said all clinical parameters, if yes, determining said rule as the target rule; or
for each physiological structure: analyzing data, which correspond to clinical parameters that are comprised in all rule(s) that is(are) associated with said physiological structure, so as to determine the target rule.

6. The method according to claim 1, **characterized in that**, the adjacent position of the abnormal waveform segment comprises a position that is located above and adjacent to the abnormal waveform segment, or a position that is located below and adjacent to the abnormal waveform segment.

7. The method according to claim 1, **characterized in that**, the abnormal indicator, which corresponds to at least one of the several clinical parameters analyzed that is comprised in the target rule, is further associated with a threshold of said at least one clinical parameter; and/or the abnormal indicator, which corresponds to at least one of the several clinical parameters analyzed that is comprised in the target rule, is further associated with a waveform morphology of said at least one clinical parameter.

8. The method according to claim 1, **characterized in that**:
when the physiological system comprises the nervous system, a plurality of clinical parameters that are associated with the nervous system are a plurality of clinical parameters that are associated with a brain nerve;
when the physiological system comprises the circulatory system, a plurality of clinical parameters that are associated with the circulatory system are a plurality of clinical parameters that are associated with hemodynamics or perfusion;
when the physiological system comprises the respiratory system, a plurality of clinical parameters that are associated with the respiratory system are a plurality of clinical indicators that are associated with oxygenation.

9. The method according to claim 1, **characterized in that**:
an abnormal state, which is pre-associated with a rule that is pre-associated with the circulatory system, is shock; wherein said rule comprises an abnormal indicator that is associated with a trend of change for a PI parameter or a trend of change for a CQI parameter, and an abnormal indicator that is associated with a trend of change for a blood pressure; and/or
an abnormal state, which is pre-associated with a rule that is pre-associated with the circulatory system, is a circulatory deterioration; wherein said rule comprises an abnormal indicator that is associated with a trend of change for a heart rate, and an abnormal indicator that is associated with a trend of change for blood oxygen; and/or
an abnormal state, which is pre-associated with a rule that is pre-associated with the circulatory system, is a circulatory instability; wherein said rule comprises an abnormal indicator of an electrocardiogram signal, and an abnormal indicator that is associated with a trend of change for blood oxygen; and/or
an abnormal state, which is pre-associated with a rule that is pre-associated with the respiratory system, is a respiratory distress; wherein said rule comprises an abnormal indicator that is associated with a trend of change for a heart rate, and an abnormal indicator that is associated with a trend of change for a respiratory rate.

10. The method according to claim 1, **characterized in that**, further comprising:
when the abnormal state of the physiological structure that corresponds to the target rule comprises RonT arrhythmia or QTc prolongation, obtaining dosage data infused to the patient within a preset time range and displaying a waveform of the dosage data on the display interface.

11. A method for processing vital information that is applicable to a system for processing vital information, **characterized in that**, the system for processing vital information comprises a memory, a processor and a display, wherein the memory is configured to store an executable program, the processor is configured to execute the executable program, so as to enable the processor to implement following operations:
obtaining patient data of a patient;
analyzing the patient data to obtain an abnormal state of the patient and a waveform of the patient data that are associated with the abnormal state of the patient; wherein the waveform of the patient data has an abnormal waveform segment that is associated with the abnormal state of the patient; the abnormal state of the patient comprises an abnormal state of at least one physiological structure of the patient, wherein the physiological structure at least comprises a physiological system, wherein the physiological system comprises at least one of: a nervous system, a circulatory system or a respiratory system, of the patient; and
controlling a display interface of the display to display state information that represents the abnormal state of the patient, and the waveform of the patient data, wherein the state information comprises graphic and text that explain the abnormal waveform segment; wherein displaying specifically comprises associatively displaying the graphic and the text with the abnormal waveform segment, wherein the graphic at least comprises an arrow that represents a trend of change for the patient data; wherein associatively displaying the graphic and the text with the abnormal waveform segment, comprises: displaying the arrow on or adjacent to the abnormal waveform segment; wherein:
when the physiological system comprises the nervous system of the patient, the text is configured to describe an abnormal state of an indicator that is associated with a brain nerve;
when the physiological system comprises the circulatory system of the patient, the text is configured to describe an abnormal state of an indicator that is associated with hemodynamics or perfusion;
when the physiological system comprises the respiratory system of the patient, the text is configured to describe an abnormal state of an indicator that is associated with oxygenation.

12. The method according to claim 11, **characterized in that**, analyzing the patient data to obtain an abnormal state of the patient, comprises:
analyzing the patient data that are associated with the abnormal state of the patient, so as to determine one or more target rules that are satisfied by the patient data, and obtaining the abnormal state of the patient, which corresponds to the one or more target rules that are satisfied by the patient data;
wherein the graphic and the text correspond to the one or more target rules that are satisfied by the patient data.

13. The method according to claim 12, **characterized in that**, one target rule comprises one or more indicators;
determining whether the patient data satisfy one target rule, comprises:
determining whether the patient data satisfy one or more indicators that are comprised in said one target rule, if yes, determining that the patient data satisfy said one target rule, if not, determining that the patient data does not satisfy said one target rule;
wherein when the physiological system comprises the nervous system of the patient, the one or more indicators comprise indicator(s) that is(are) associated with a brain nerve;
when the physiological system comprises the circulatory system of the patient, the one or more indicators comprise indicator(s) that is(are) associated with hemodynamics or perfusion,
when the physiological system comprises the respiratory system of the patient, the one or more indicators comprise indicator(s) that is(are) associated with oxygenation.

14. The method according to claim 13, **characterized in that**, associatively displaying the graphic and the text with the abnormal waveform segment, comprises:
displaying first text that corresponds to a first indicator adjacent to a first graphic that corresponds to the first indicator;
displaying second text that corresponds to a second indicator adjacent to a second graphic that corresponds to the second indicator;
wherein, the first indicator and the second indicator are two indicators that are satisfied by the patient data.

15. The method according to claim 13, **characterized in that**, determining whether the patient data satisfy one or more indicators that are comprised in said one target rule, comprises:
obtaining a first comparison result between the patient data and a preset data threshold, and determining whether the patient data satisfy the one or more indicators that are comprised in said one target rule based on the first comparison result; and/or
obtaining the trend of change for the patient data, and determining whether the patient data satisfy the one or more indicators that are comprised in said one target rule based on the trend of change; and/or
obtaining a second comparison result between the waveform of the patient data and a preset waveform template, and determining whether the patient data satisfy the one or more indicators that are comprised in said one target rule based on the second comparison result.

16. The method according to claim 15, **characterized in that**, associatively displaying the graphic and the text with the abnormal waveform segment, further comprises:
displaying the first comparison result between patient data corresponding to the abnormal waveform segment and the preset data threshold; and/or
marking, on or adjacent to the abnormal waveform segment, the trend of change for patient data corresponding to the abnormal waveform segment; and/or
comparing and displaying the abnormal waveform segment with a corresponding preset waveform template; and/or
marking the abnormal waveform segment through the graphic and the text.

17. The method according to claim 16, **characterized in that**, marking, on or adjacent to the abnormal waveform segment, the trend of change for patient data corresponding to the abnormal waveform segment, at least comprises:
displaying patient data before change at or adjacent to a start position of the abnormal waveform segment, and displaying patient data after change at or adjacent to an end position of the abnormal waveform segment.

18. The method according to any one of claims 11-17, **characterized in that**, controlling a display interface of the display to display state information that represents the abnormal state of the patient, and the waveform of the patient data, comprises:
providing a state analysis window, and displaying the state information and the waveform of the patient data within the state analysis window;
wherein the state analysis window has any one of following characteristics:
displaying the state analysis window on the display interface for a long term;
automatically displaying the state analysis window on the display interface, when the abnormal state of the patient is obtained; or
prompting a user, on the display interface, to input an operation to open the state analysis window, when the abnormal state of the patient is obtained, and displaying the state analysis window on the display interface, after the operation to open the state analysis window is detected.

19. The method according to claim 18, **characterized in that**, the monitoring data comprises real-time monitoring data that are currently monitored;
the method further comprises:
generating real-time alarm information based on the real-time monitoring data, and displaying the real-time alarm information and the state analysis window in different areas of the display interface.

20. The method according to claim 18, **characterized in that**, prompting a user, on the display interface, to input an operation to open the state analysis window, when the abnormal state of the patient is obtained, comprises:
displaying a designated icon at a fixed position on the display interface for a long term; and changing a display mode of the designated icon, when the abnormal state of the patient is obtained, so as to prompt the user to trigger the designated icon to open the state analysis window; or
automatically displaying a designated icon on the display interface, when the abnormal state of the patient is obtained, so as to prompt the user to trigger the designated icon to open the state analysis window.

21. The method according to claim 20, **characterized in that**, the designated icon displayed is in a one-to-one correspondence with at least one physiological structure; wherein, when the designated icon is triggered, the designated icon is configured to display, in the state analysis window, the graphic, the text and the waveform of the patient data, that are associated with the abnormal state of a corresponding physiological structure.

22. The method according to claim 19, **characterized in that**, the state analysis window is further configured to display a tag that is in a one-to-one correspondence with at least one physiological structure;
when the tag is triggered, the state analysis window is configured to display, in the state analysis window, the graphic, the text and the waveform of the patient data, that are associated with the abnormal state of a corresponding physiological structure.

23. The method according to claim 19, **characterized in that**, the display interface is further configured to display a content that is different from the state analysis window;
when the state analysis window is displayed on the display interface for a long term, the state analysis window and the content are displayed independently and completely in different areas of the display interface;
when the state analysis window is automatically displayed on the display interface, or when the state analysis window is displayed on the display interface based on the operation to open the state analysis window, at least part of the content is not blocked by the state analysis window, or a layout and/or a size of the content are/is adaptively adjusted to be completely displayed on the display interface.

24. The method according to claim 11, **characterized in that**, the abnormal state of the patient further comprises at least one of: an overall abnormal state, an abnormal state of an organ, an abnormal state of a physiological part, or an abnormal state of a tissue, of the patient.

25. The method according to any one of claims 11-24, **characterized in that**, the physiological system further comprises at least one of: a motor system, an endocrine system, a digestive system, an urinary system, or a reproductive system, of the patient.

26. The method according to any one of claims 11-25, **characterized in that**, the physiological structure further comprises a physiological organ, a physiological part, a tissue, a physiological system, a feature of a physiological system, or a feature of a physiological organ;
the abnormal state of the patient further comprises at least one of: an overall abnormal state of the patient, an abnormal state of the physiological organ, an abnormal state of the physiological part, an abnormal state of the tissue, an abnormal state of the feature of the physiological system, or an abnormal state of the feature of the physiological organ.

27. A method for processing vital information that is applicable to a system for processing vital information, **characterized in that**, comprising:
obtaining patient data of a patient;
analyzing the patient data to obtain an abnormal state of the patient and a waveform of the patient data that are associated with the abnormal state of the patient; wherein the waveform of the patient data has an abnormal waveform segment that is associated with the abnormal state of the patient; and
controlling a display interface to display state information that represents the abnormal state of the patient, and the waveform of the patient data, and providing graphic and text that explain the abnormal waveform segment; wherein the graphic and the text are associatively displayed with the abnormal waveform segment.

28. A system for processing vital information, **characterized in that**, comprising: a memory, a processor and a display, wherein the memory is configured to store an executable program, the processor is configured to execute the executable program, so as to enable the processor to implement the method according to any one of claims 11-26.

29. A computer-readable storage medium, **characterized in that**, comprising a program that is executable by a processor to implement the method according to any one of claims 11-26.
